## Europäisches Patentamt

## European Patent Office

## Office européen des brevets

(19)

(11) Veröffentlichungsnummer: **0 099 592**
**B1**

(12)

# EUROPÄISCHE PATENTSCHRIFT

(45) Veröffentlichungstag der Patentschrift:
**10.12.86**

(51) Int. Cl.⁴: **C 07 D 213/06**

(21) Anmeldenummer: **83200526.8**

(22) Anmeldetag: **13.04.83**

(54) **Verfahren zur katalytischen Dehydrierung von Piperidin.**

(30) Priorität: **20.07.82 DE 3227022**

(43) Veröffentlichungstag der Anmeldung:
**01.02.84 Patentblatt 84/5**

(45) Bekanntmachung des Hinweises auf die Patenterteilung:
**10.12.86 Patentblatt 86/50**

(84) Benannte Vertragsstaaten:
**BE CH DE FR GB IT LI NL**

(56) Entgegenhaltungen:
**DE - B - 1 192 648**
**DE - C - 579 146**
**DE - C - 942 990**

(73) Patentinhaber: **Rütgerswerke Aktiengesellschaft,
Mainzer Landstrasse 217, D-6000 Frankfurt a.Main 1 (DE)**

(72) Erfinder: **Grigoleit, Georg, Dr., Moselweg 7,
D-4270 Dorsten-19 (DE)**
Erfinder: **Oberkobusch, Rudolf, Dr., Am Botanischen
Garten 3, D-4100 Duisburg-1 (DE)**
Erfinder: **Stadelhofer, Jürgen, Dr.,
Groppenbrucherstrasse 121, D-4600 Dortmund-15 (DE)**
Erfinder: **Matern, Kurt, Quadtstrasse 9,
D-4100 Duisburg-12 (DE)**

## Beschreibung

Die vorliegende Erfindung bezieht sich auf ein verbessertes Verfahren zur Herstellung von Pyridin durch katalytische Dehydrierung von Piperidin in der Gasphase.

Pyridin und Pyridinbasen sind wichtige Zwischenverbindungen für die organische Chemie und insbesondere die Agrochemie und pharmazeutische Chemie. Abgesehen von der Gewinnung von Pyridin auf steinkohlenteerstämmiger Basis, haben die bislang hauptsächlich praktizierten synthetischen Verfahren die Nachteile, dass sie teils auf teuren petrochemischen Rohstoffen beruhen und dass andererseits neben Pyridin eine Reihe nicht immer erwünschter alkylierter Pyridine als Koppelprodukte anfallen.

Als von petrostämmigen Rohstoffen unabhängiger Rohstoff für die Pyridinherstellung bietet sich Tetrahydrofurfurylalkohol an, der durch Hydrierung von Furfurylaldehyd herstellbar ist.

Furfurylaldehyd wird aus landwirtschaftlichen Abfallprodukten, in denen Pentosane, die hauptsächliche Vorläufer für Furfurylaldehyd sind, gewonnen. Durch Umsetzung von Ammoniak mit Tetrahydrofurfurylalkohol lässt sich Piperidin herstellen, das als Ausgangsprodukt für das hier beschriebene Verfahren dient.

Für die Herstellung von Pyridin aus Piperidin sind mehrere Verfahren bekannt. Sie unterscheiden sich im wesentlichen in den zur Anwendung gelangenden Katalysatoren und den Reaktionstemperaturen, die zwischen 200 und 600°C liegen, sowie in der Menge des bei dem Prozess als Trägergas angewandten Wasserstoffs, die im Wasserstoff-Piperidin-Molverhältnis angegeben wird. Diese offenbarten Molverhältnisse reichen von 4,8:1 bis etwa 18:1.

So lehren DE-PS 1 192 648 und GB-PS 745 400 die Gasphasendehydrierung mit Pt oder Pd als Katalysator. Obwohl die Angaben über Umsatz und Selektivität bestechend sind, wurde gefunden, dass die Edelmetallkatalysatore doch empfindlich gegenüber Kontaktgiften wie Schwefel, Halogenen oder Ammoniak sind, so dass ihre Wirksamkeit nach kurzer Zeit nachlässt. Vor allem nach einer Reaktivierung dieses Katalysators tritt sehr schnell ein Aktivitätsverlust ein.

Gemäss DE-PS 579 146 wird die Dehydrierung von heterocyclischen Verbindungen durch Metalle der sechsten Gruppe des periodischen Systems, die auch mit anderen Metallen wir Zink, Kupfer, Kobalt, Nickel, Calcium oder Magnesium vermischt sein können, katalysiert. Im offenbarten Beispiel der Dehydrierung von Piperidin dient Wolframoxid als Katalysator. Allerdings wird bei 410°C nur ein Umsatz von 84% erzielt und dabei noch ein hoher Prozentsatz (10%) an unerwünschten Kondensationsprodukten erhalten.

Ähnliche, ebenfalls unbefriedigende Ergebnisse hinsichtlich Reaktivität und Selektivität bei der Herstellung von Pyridin aus Piperidin ergibt das in Z. absc. Chim. 29 (1959) 440–443 engl. Ausgabe beschriebene Katalysatorsystem Chrom/Kupfer. Bei einer Reaktionstemperatur von 400°C wird zwar ein Piperidinumsatz von 86% erreicht, aber nur eine Pyridin-Selektivität von 53,2%.

Es bestand daher die Aufgabe, ein Verfahren zur katalytischen Dehydrierung von Piperidin zu Pyridin zu finden, bei dem neben einem hohen Umsatz und einer hohen Selektivität zu Pyridin lange Katalysatorstandzeiten erreicht werden, so dass eine wirtschaftliche Pyridingewinnung über diese Route ermöglicht wird.

Diese Aufgabe wird gelöst durch ein Verfahren gemäss der Ansprüche 1–6.

Es wurde gefunden, dass bei der Dehydrierung von Piperidin bei hohem Umsatz eine Pyridin-Selektivität von 97% erreicht werden kann, wenn als Katalysator ein Siliciumdioxid verwendet wird, das mit Kupfer, Nickel und Chrom in einer Menge 5–12:1–5:0,1–<0,7 Gew.-% aktiviert ist. Besonders gute Umsetzungen werden bei einem Verhältnis der Metalle von 9:3:0,6 erhalten.

Im Gegensatz zur Lehre der DE-PS 579 146 wurde gefunden, dass bei einem Katalysator mit hohem Umsatz und hoher Selektivität zu Pyridin das Metall der sechsten Gruppe des periodischen Systems lediglich in Spuren bis zu weniger als 0,7 Gew.-% anwesend sein darf. Ausserdem ist das Trägermaterial von Bedeutung. So zeigte es sich, dass bessere Ausbeuten mit Siliciumdioxid anstelle von Aluminiumoxid als Trägermaterial erzielt werden.

Frische Katalysatoren zeigen erwartungsgemäss die grösste Aktivität und wirken bereits bei Temperaturen ab 300°C dehydrierend. Während des Gebrauchs der Katalysatoren bildet sich auf diesen durch Zersetzungsreaktionen ein Kohlenstoffbelag, der die Reaktivität des Katalysators reduziert. Um dies auszugleichen, wird im Verlaufe einer längeren Gebrauchsdauer des Katalysators die Reaktionstemperatur erhöht. Im erfindungsgemässen Verfahren erfolgt die Dehydrierung bei Temperaturen zwischen 300 und 420°C, bevorzugt zwischen 345 und 380°C. Es hat sich gezeigt, dass durch zunehmenden Kohlenstoffbelag auf dem Katalysator sich zwar seine Reaktivität vermindert, sich aber die Selektivität zu Pyridin verbessert. Optimale Bedingungen werden bei einem Kohlenstoffgehalt von 1–2% erzielt. Steigt der Kohlenstoffgehalt auf 5–6%, so sinkt die Reaktivität derart, dass der Katalysator reaktiviert werden muss. Dies erfolgt in bekannter Weise durch Oberleiten von Sauerstoff bei Temperaturen von 400 bis 440°C und nachfolgende Behandlung mit Wasserstoff bei Temperaturen von 150 bis 350°C.

Die Dehydrierung von Piperidin kann mit nur einer Katalysatorfüllung kontinuierlich über Monate durchgeführt werden, wenn man bei Eintreten einer Aktivitätsminderung des Katalysators den abgelagerten Kohlenstoff jeweils wieder bis auf 1–2% reduziert. Diese sehr langen Katalysatorstandzeiten sind ein wesentlicher Vorteil des erfindungsgemässen Katalysatorsystems.

Das zu dehydrierende Piperidin wird in einer dem Reaktor vorgeschalteten Verdampfervorrichtung verdampft und dann mit Wasserstoff als Trägergas über den Katalysator geleitet.

Als optimale Wasserstoffmenge wurde ein Wasserstoff/Piperidin-Molverhältnis von 5:1 ermittelt. Bei diesem Verhältnis ist z.B. der Piperidinumsatz fast um 20% höher als bei einem Molverhältnis von 3:1. Andererseits führt eine weitere Erhöhung des Wasserstoffangebots auf ein Molverhältnis von z.B. 8:1 zu keiner weiteren Umsatzsteigerung, wohl aber wird durch den hohen Wasserstoffanteil die Raum/Zeit-Ausbeute reduziert.

Als Wasserstoff kann sowohl frischer Wasserstoff als auch das bei der Dehydrierung anfallende, hauptsächlich Wasserstoff enthaltende Konversionsgas eingesetzt werden. Voraussetzung für die Verwendung des Konversionsgases ist dessen Reinigung und gute Trocknung.

Dazu wird der Teil des bei der Dehydrierung anfallenden Gasgemisches, der in den Reaktor zurückgeführt werden soll, mit verdünnter Schwefelsäure gewaschen, gut getrocknet und über ein Molekularsieb geleitet. Abgesehen davon, dass es wirtschaftlich günstiger ist, das Konversionsgas aus der Dehydrierungsreaktion im Kreislauf zu fahren, anstatt während des gesamten Verfahrens frischen Wasserstoff zu verwenden, ist es ausserordentlich überraschend, dass durch die erfindungsgemässe Reinigung und Rückführung des Konversionsgases in den Reaktor der Piperidin-Umsatz gegenüber dem Einsatz von frischem Wasserstoff um mehr als 5% gesteigert werden kann, wobei sich allerdings die Selektivität etwas zugunsten von α-Picolin verschiebt. Vermutlich erfolgt durch im gereinigten Konversionsgas noch vorhandene Spuren anderer Gase wie z.B. Methan oder Kohlenmonoxid ein zusätzlicher katalytischer Effekt.

Die folgenden Beispiele und Vergleichsbeispiele verdeutlichen die erfindungsgemässen Verfahrensdurchführungen. Dabei sind die angegebenen Ausbeuten, Umsätze und Selektivitäten wie folgt definiert:

$$\text{Ausbeute:} \quad \frac{\text{Menge gebildetes Pyridin}}{\text{Menge max. erwartetes Pyridin}} \times 100$$

$$\text{Umsatz:} \quad \frac{\text{Mol umgesetzte Verbindung}}{\text{Mol zugeführte Verbindung}} \times 100$$

$$\text{Selektivität:} \quad \frac{\text{Mol erhaltenes Produkt}}{\text{Mol umgesetzte Verbindung}} \times 100$$

Alle Versuche wurden in einem Glasrohr-Reaktor, der aus einer Vorheizzone und dem eigentlichen Reaktorteil besteht, durchgeführt. Der Reaktor hat eine Länge von 800 mm und einen Durchmesser von 20 mm. Der Vorheizteil ist 350 mm lang und hat einen Durchmesser von 25 mm. Verdampft wird das Piperidin aus einem 250 ml Quarzkolben. Die Beheizung des Reaktors und Verdampfers erfolgt durch Heissluft, die des Vorerhitzers elektrisch. Die Kontaktfüllung beträgt 55 g (100 ml). Die Körnung des Kontaktes liegt zwischen 3 und 6 mm Durchmesser. Bedingt durch jeweils bereits vorher durchgeführte Dehydrierungsreaktionen oder durch Regenerationen haben die angegebenen Katalysatore, soweit nicht anders vermerkt, bei dem jeweiligen Versuchsbeginn einen Kohlenstoffgehalt von 1–2%.

Beispiel 1

Mittels einer Dosierpumpe werden 24,3 g Piperidin/h in den Verdampferkolben gespeist. Gleichzeitig werden 32,0 l frischer Wasserstoff/h zugeleitet und das Gemisch verdampft (Wasserstoff/Piperidin-Molverhältnis 5:1). Das vorliegende Gasgemisch wird in der Vorheizzone des Reaktors auf 380°C erhitzt. Von hier gelangt es in den auf 350°C erhitzten Kontaktraum. Nach Durchlaufen des Reaktors wird das Reaktionsprodukt kondensiert. Das Konversionsgas mit dem überschüssigen Wasserstoff wird abgeleitet.

Katalysator: Siliciumdioxid mit 9% Cu, 3% Ni und 0,6% Cr. Das kondensierte Produkt ist Pyridin mit geringen Mengen an α-Picolin und Spuren anderer Pyridinbasen. Daneben enthält das Kondensat noch nicht umgesetztes Piperidin. Das Pyridin und die anderen Pyridinbasen können nach bekannten, geeigneten Verfahren aus dem Kondensat gewonnen werden, z.B. durch fraktionierte Destillation. Das hierbei anfallende Piperidin wird erneut dem Reaktor zur Dehydrierung zugeführt.

Ergebnis: Piperidinumsatz: 84,5%
Ausbeute an Pyridin: 79,9%
Selektivität zu Pyridin: 93,1%
Selektivität zu α-Picolin: 0,6%

Nach einer Versuchsdauer von 60 h haben sich der Kohlenstoffgehalt und die Aktivität des Katalysators nicht wesentlich geändert. Auch nach mehrfacher Reaktivierung des Katalysators ändert sich seine Aktivität nicht.

Beispiel 2

Die Dehydrierung wird analog Beispiel 1 durchgeführt, jedoch wird anstelle von frischem Wasserstoff als Trägergas Konversionsgas (über 90% Wasserstoff) verwendet, das sich bei einer vorangegangenen Dehydrierung gebildet hatte. Dieses Gas wird nach Reinigung mit 5%iger Schwefelsäure, Trocknung mit Calciumchlorid und Aluminiumoxid durch eine mit Molekularsieb 10A gefüllte Kolonne geleitet und danach im Kreislauf unter Einhaltung des Piperidin/Gas-Verhältnisses dem Verdampferkolben zugeführt. Das Überschussgas wird abgeleitet.

Ergebnis: Piperidinumsatz: 89,9%
Ausbeute an Pyridin: 81,1%
Selektivität zu Pyridin: 88,8%
Selektivität zu α-Picolin: 4,1%

Nach einer Versuchsdauer von 60 h haben sich der Kohlenstoffgehalt und die Aktivität des Katalysators nicht wesentlich geändert.

Beispiel 3

Die Dehydrierung von Piperidin wird analog Beispiel 1 durchgeführt, jedoch wird ein Wasserstoff/Piperidin-Molverhältnis von 3:1 verwendet.

Ergebnis: Piperidinumsatz: 76,8%
Ausbeute an Pyridin: 72,7%
Selektivität zu Pyridin: 94,7%
Selektivität zu α-Picolin: 0,9%

Beispiel 4

Die Dehydrierung von Piperidin wird analog Beispiel 1 durchgeführt, jedoch mit folgendem Katalysator:

$Al_2O_3$ mit 9% Cu, 3% Ni und 0,6% Cr
Ergebnis: Piperidinumsatz: 57,9%
Selektivität zu Pyridin: 78,4%

Beispiel 5

Die Dehydrierung von Piperidin wird analog Beispiel 1 durchgeführt, jedoch mit folgendem Katalysator:

Siliciumdioxid mit 9% Cu, 3% Ni und 0,7% Cr
Ergebnis: Piperidinumsatz: 61,6%
Selektivität zu Pyridin: 88,5%

Beispiel 6 (Vergleichsbeispiel)

Die Dehydrierung von Piperidin wird analog Beispiel 1 durchgeführt, jedoch mit folgendem Katalysator:

Siliciumdioxid mit 22% Cu, 2% $Cr_2O_3$ und 8% $BaCrO_4$
Ergebnis: Piperidinumsatz: 11,3%
Selektivität zu Pyridin: 85,4%

Beispiel 7 (Vergleichsbeispiel)

Die Dehydrierung von Piperidin wird analog Beispiel 1 durchgeführt, jedoch mit folgendem Katalysator:

0,3% Pd auf $Al_2O_3$ (der Katalysator wird in frischem Zustand eingesetzt)

Ergebnis: Piperidinumsatz: 81,4%
Ausbeute an Pyridin: 74,7%
Selektivität zu Pyridin: 91,8%
Selektivität zu α-Picolin: 2,9%

Nach der ersten Reaktivierung des Katalysators wird ein sehr schneller Abbau der Katalysatoraktivität beobachtet, so dass bereits nach einer Reaktionszeit von nur 18 h der Piperidinumsatz auf 43,4% sinkt.

## Patentansprüche

1. Verfahren nach katalytischen Dehydrierung von Piperidin zu Pyridin in der Gasphase unter Verwendung von Wasserstoff als Trägergas, dadurch gekennzeichnet, dass als Katalysator ein mit Kupfer, Nickel und Chrom aktiviertes Siliciumdioxid verwendet wird, wobei die aktivierenden Metalle Kupfer, Nickel und Chrom in einer Menge von 5–12:1–5:0,1–<0,7 Gewichtsteilen pro 100 Gewichtsteilen Katalysator vorliegen.

2. Verfahren nach Anspruch 1, dadurch gekennzeichnet, dass die aktivierenden Metalle Kupfer, Nickel und Chrom in 9:3:0,6 Gewichtsteilen pro 100 Gewichtsteilen Katalysator vorliegen.

3. Verfahren nach Anspruch 1 und 2, dadurch gekennzeichnet, dass als Trägergas getrocknetes und gereinigtes Konversionsgas aus der Dehydrierungsreaktion von Piperidin zu Pyridin verwendet wird.

4. Verfahren nach Ansprüchen 1 bis 3, dadurch gekennzeichnet, dass das Wasserstoff/Piperidin-Molverhältnis 5:1 beträgt.

5. Verfahren nach Ansprüchen 1 bis 4, dadurch gekennzeichnet, dass die Dehydrierungsreaktion im Temperaturbereich von 345–380° durchgeführt wird.

6. Verfahren nach Ansprüchen 1 bis 5, dadurch gekennzeichnet, dass der Kohlenstoffbelag des Katalysators 1 bis 2 Gew.% des Katalysatorgewichts beträgt.

## Claims

1. A process for the catalytic dehydrogenation of piperidine in the gas phase using hydrogen as the carrier gas, characterized in, that as catalyst is used a silicon dioxide activated with copper, nickel and chromium as the catalyst, the activating metals copper, nickel and chromium being present in an amount of 5–12:1–5:0.2–0.7 parts by weight per 100 parts by weight of catalyst.

2. The process as claimed in claims 1, wherein the activating metals copper, nickel and chromium are present in an amount of 9:3:0.6 parts by weight per 100 parts by weight of the catalyst.

3. The process as claimed in claims 1 and 2, wherein as carrier gas a dried and purified conversion gas is used which derives from the dehydrogenation reaction of piperidine to pyridine.

4. The process as claimed in claims 1 to 3, wherein the hydrogen/piperidine molar ratio is 5:1.

5. The process as claimed in claims 1 to 4, wherein the dehydrogenation reaction is carried out in the temperature range from 345° to 380°C.

6. The process as claimed in claims 1 to 5, wherein the carbon deposit on the catalyst amounts to 1 to 2% by weight of the catalyst weight.

## Revendications

1. Procédé pour la déshydrogénation catalytique de la pipéridine en pyridine en phase gazeuse en utilisant de l'hydrogène comme gaz porteur, caractérisé en ce qu'on utilise comme catalyseur un dioxyde de silicium activé par le cuivre, le nickel et le chrome, les métaux activants cuivre, nickel et chrome étant présents en une quantité de 5–12:1–5:0,1–<0.7 parties en poids pour 100 parties en poids de catalyseur.

2. Procédé selon la revendication 1, caractérisé en ce que les métaux activants cuivre, nickel et chrome sont présents à raison de 9:3:0,6 parties en poids pour 100 parties en poids de catalyseur.

3. Procédé selon la revendication 1 et 2, caractérisé en ce qu'on utilise comme gaz porteur du gaz de conversion séché et purifié provenant de la réaction de déshydrogénation de la pipéridine en pyridine.

4. Procédé selon les revendications 1 à 3, caractérisé en ce que le rapport molaire hydrogène/pipéridine est de 5:1.

5. Procédé selon les revendications 1 à 4, caractérisé en ce que la réaction de déshydrogénation est effectuée dans le domaine de température de 345–380°.

6. Procédé selon les revendications 1 à 5, caractérisé en ce que la teneur en carbone du catalyseur est de 1 à 2% en poids du poids de catalyseur.